# EUROPÄISCHE PATENTSCHRIFT

(11) **EP 1 066 837 B1**
(45) Veröffentlichungstag und Bekanntmachung des Hinweises auf die Patenterteilung: **21.02.2007**
(21) Anmeldenummer: 00890211.6
(22) Anmeldetag: 05.07.2000
(51) Int. Cl.: A61L 2/12, A61L 11/00

(54) **Mikrowellensterilisationseinrichtung**
Microwave sterilisation device
Dispositif à Micro-ondes pour stérilisation

(30) Priorität: 07.07.1999 AT 117999
(43) Veröffentlichungstag der Anmeldung: 10.01.2001
(73) Patentinhaber: Katschnig, Helmut, Dr., A-8750 Judenburg Steiermark (AT)
(72) Erfinder: Katschnig, Helmut, Dr., 8750 Judenburg (AT); Stegmüller, Wolfgang, 8755 St. Peter/Judenburg (AT); Gruber, Ernst, 8750 Judenburg (AT)
(74) Vertreter: Itze, Peter

(56) Entgegenhaltungen:
- EP-A- 0 483 104
- EP-A- 0 759 304
- WO-A-90/12601
- WO-A-96/38021

## Beschreibung

Die Erfindung betrifft sich auf eine Anlage zum Trocknen, Erhitzen, Sterilisieren und/oder Desinfizieren von feuchten, befeuchteten oder in feuchter Umgebung befindlichen Gütern sowie von Flüssigkeiten, insbesondere von Abfällen, die gegebenenfalls infiziert sind, z.B. von medizinischen Abfällen, bei welcher die zu behandelnden Güter in einem mikrowellendurchlässigen Behälter in einen mit Mikrowellen beaufschlagbaren, druckdicht abschließbaren, druckfesten Behandlungsraum eingebracht werden, wobei mehrere Magnetrone so in den den Behandlungsraum bildenden Resonanzraum eingekoppelt sind, daß mikrowellentote Räume vermieden sind, wobei die physikalischen Parameter im Gerät über an mit dem Behälterinnenraum kommunizierenden Trägem angeordneten Einrichtungen überwacht werden, und gegebenenfalls zur Steuerung der Anlage herangezogen werden, und wobei noch Zusatzaggregate zur Beinflussung des Verfahrensablaufes vorgesehen sind, sowie auf Verfahren zum Pasteurisieren, Desinfizieren/Sterilisieren von feuchten, befeuchteten oder in feuchter Umgebung befindlichen Gütern sowie von Flüssigkeiten.

Eine derartige Anlage geht aus WO96/38021 hervor, bei welcher in einem zylindrischen Druckkessel ein Innenbehälter zur Aufnahme eines mit dem zu desinfizierenden Gut gefüllten Behälters vorgesehen ist. Der Druckkessel ist etwa mittig entlang einer Querebene geteilt, wobei auch der Innenbehälter entlang der gleichen Ebene geteilt und entlang der freien Kanten mit dem zugehörigen Druckkesselteil verbunden ist. In den Innenbehälter, dessen Innenraum über Öffnungen mit dem Innenraum des Druckkessels kommuniziert, sind an der Mittelachse endseitig Mikrowellengeneratoren eingekoppelt, wobei die Wandungen des Innenbehälters zur Fokussierung der Mikrowellen dienen. Zur Beschickung ist der obere Teil des Druckkessels zusammen mit dem oberen Teil des Innenbehälters abzuheben, dann der mit dem zu desinfizierenden Gut gefüllte Behälter von oben her in den unteren Teil des Innenbehälters und damit den unteren Teil des Druckkessels einzusetzen und danach der obere Teil der Einrichtung über dem unteren Teil zu positionieren und abzusenken. Danach werden beide Teile gegeneinander druckdicht verriegelt.

Eine andere bekannte Anlage geht z.B. auch aus WO 86/02842 hervor, bei welcher ein zylindrischer Innenraum vorgesehen ist, der internen Drücken bis etwa 5 Atmosphären oder einem teilweisen Vakuum widerstehen kann, welcher zylindrische Raum an seiner Oberseite durch einen Deckel verschließbar ist. Die Magnetrone sind an der unteren Stirnseite des zylindrischen Behälters vorgesehen, welche Unterseite durch eine mikrowellendurchlässige druckfeste Wandung abgeschlossen ist. Um bei dieser bekannten Ausbildung eine möglichst gleichmäßige Verteilung der Mikrowellenenergie zu erzielen, ist die innere zylindrische Kammer drehbar aufgehängt, um so aufgrund der Bewegung dieser zylindrischen Kammer eine Steuerung der Bestrahlungsdichte der einzelnen Teilchen zu erreichen. Überdies ist aufgrund dieser Ausbildung die Kammer der bekannten Ausführung nur von oben her beschickbar, was rasch dann zu Schwierigkeiten führen kann, wenn in den Behälter größere Mengen von in einem Gebinde befindlichem Gut einzubringen sind. Weiters kann sich bei dieser bekannten Ausbildung an der Wandung Kondenswasser beim Abkühlen bilden, welches aus dem Behälter entfernt werden muß.

Eine weitere bekannte Anlage geht aus EP 0 476 004 B1 hervor, bei welcher gleichfalls eine oben verschließbare zylindrische Behandlungskammer vorgesehen ist, wobei diese Behandlungskammer (dort Hohlraumresonator genannt) durch ein gasdicht verschließbares Gefäß 1 ausgekleidet ist und wobei die Magnetrone seitlich und von der unteren Stirnfläche her in den Behandlungsraum eingekoppelt sind. Durch die gemäß dieser bekannten Ausbildung vorgesehene Auskleidung wird der Abstand des Innenraumes von den Mikrowellengeneratoren so gesteuert, daß auch in diesem Außenbereich eine entsprechende Energiedichte vorhanden ist.

Auch diese bekannte Ausbildung hat den Nachteil, daß sie von oben her beschickbar ist, was das Beschicken entsprechend erschwert. Außerdem ist, wie dieser Druckschrift entnehmbar ist, das Gerät mit entkalktem Wasser zu beschicken, da es sonst zu Ablagerungen von Kalk in der Auskleidung kommen würde. Auch die Ausbildung gemäß dieser Druckschrift ist in der Lage, mit Überdruck und/oder Unterdruck zu arbeiten.

Auch bei der Ausbildung gemäß WO90/12601 ist ein nur von oben beschickbares Behandlungsgefäß vorgesehen, welches mit einem Mikrowellenheizsystem versehen ist, wobei die Einkoppelung der Mikrowellen nicht näher beschrieben ist. Bei dieser bekannten Ausbildung ist zusätzlich die Einbringung von Ozon oder Inertgas wie Stickstoff vorgesehen.

Aus EP 0 287 549 Bl, deren Gegenstand auch auf eine Erfindung des Anmelders der vorliegenden Anmeldung als Miterfinder zurückgeht, ist es bekannt, in einen Hohlresonator mehrere Magnetrone derart einzukoppeln, daß mikrowellentote Räume vermieden sind, wobei das Einkoppeln über Hohlleiter erfolgt. Diese bekannte Ausbildung ist allerdings nicht als Überdrucksterilisator vorgesehen.

Auch EP 0 483 104 B 1 geht auf den vorliegenden Anmelder als Miterfinder zurück, wobei in einer Anlage gemäß der vorstehend genannten EP 0 287 549 in den Hohlraumresonator, also die Behandlungskammer, ein Gefäß eingebracht wird, das infektiösen Abfall enthält, wobei dieses Gefäß mit einem Deckel abschließbar ist, sodaß dieses Gefäß als Sammelgefäß für diesen infektiösen Abfall dienen kann. Durch eine Öffnung im Deckel dieses Gefäßes ist von oben her in das Gefäß ein Träger mit physikalischen Meßsensoren einsenkbar, wobei über diesen Träger auch Wasser eingesprüht werden kann, wenn die im infektiösen Gut enthaltene Wassermenge bzw. Feuchtigkeitsmenge zu gering ist. Dies wird gemäß der bekannten Ausbildung über eine entsprechende Wiegeeinrichtung ermittelt, wobei die Steuerung der Wasserzugabe über eine zentrale Steuereinrichtung erfolgt, die die Mikrowellengeneratoren entsprechend den gemessenen physikalischen Daten steuert.

Der vorliegenden Erfindung liegt die Aufgabe zugrunde, eine Anlage der eingangs genannten Art zu schaffen, welche einerseits die Vorteile der seitlich beschickbaren Behandlungskammer gemäß EP 483 104 A2 und anderseits auch die Vorteile einer Druckkammer aufweist.

Erfindungsgemäß wird dies dadurch erreicht, daß die Magnetrone druckdicht über an sich bekannte Hohlleiter von der Behälterseitenwandung her in den Behandlungsraum eingekoppelt sind, der mit einer seitlichen Beschickungsöffnung zum Einbringen des mikrowellendurchlässigen Behälters unter Belassung eines Freiraumes zwischen Behälter und Behandlungsrauminnenwandung versehen ist, wobei der Behandlungsraum zylindrische oder polygonal prismatische Form mit vertikal verlaufender Längsachse aufweist, und wobei die Beschickungsöffnung durch Teilen der Behandlungskammer entlang einer parallel zur Längsachse der Behandlungskammer verlaufende, vorzugsweise außermittige Ebene, erzeugt ist, wobei auch die Boden- und die Deckwandung geteilt ist, oder durch Ausschneiden der Mantelwandung der Behandlungskammer entlang zweier Erzeugenden sowie parallel zur Boden- und Deckwandung erzeugt ist. Dadurch wird erreicht, daß über die Hohlleiter und den zwischen dem Behälter und der Innenwandung des Behandlungsraumes freibleibenden Zwischenraum die Distanz zwischen zu behandelndem Gut und Mikrowellengenerator so gesteuert werden kann, daß der gesamte Behälterinnenraum die volle Mikrowellenleistung erhält, sodaß für die Erzeugung des nötigen Dampfes zur Erreichung des Arbeitsüberdruckes die eingestrahlte Mikrowellenenergie ausreicht. Außerdem wird durch die seitliche Beschickungsöffnung ermöglicht, daß auch großvolumigere Behälter, die ohne Hilfsgeräte praktisch nicht mehr handhabbar wären, in den Behandlungsraum eingebracht werden können und zwar auch mit einem Hilfsmittel, sei es eine entsprechend ausgebildete Sackkarre, einen Hubwagen od.dgl. Eine Ausbildung bei der die Beschickungsöffnung durch Ausschneiden der Mantelwandung der Behandlungskammer entlang zweier Erzeugenden sowie parallel zur Boden- und Deckwandung erzeugt ist, wodurch die gesamte Boden- und Deckfläche erhalten bleibt und die Beschickungsöffnung lediglich in der Wandung vorgesehen ist, kann unter Umständen aus platzökonomischen Gründen vorteilhaft sein.

Für eine besonders raumsparende Ausbildung kann entlang der Beschickungsöffnung zum dichten Abschluß dieser Öffnung mittels des zugehörigen Abschlußteiles eine Kombination aus an sich bekannter Wellenfalle und Dichtung, vorzugsweise eine mit Innendruck beaufschlagbare Schlauchdichtung, vorgesehen sein. Dies gibt die Möglichkeit, sowohl die Abdichtung gegen Mikrowellenaustritt als auch gegen Druckaustritt auf engem Raum zu gestalten, ohne daß die freie Durchgangsöffnung beeinträchtigt wird.

Um sicherzustellen, daß der Sterilisations-/Desinfektionsvorgang auch tatsächlich bei der richtigen Temperatur innerhalb des Gutes und nicht nur der Innenraumtemperatur stattfindet, kann zur Bestimmung der Regelgröße für die Leistung der Magnetrone ein Thermofühler über den mit dem Behälterinnenraum kommunizierenden Träger in den mikrowellendurchlässigen Behälter einbringbar sein, der mit einer, einen Sollwertspeicher und einen Komparartor zum Vergleich mit dem erhobenen Istwert aufweisenden Steuereinrichtung verbunden ist. Es wird dadurch viel direkter die Temperatur im Gut erfaßt, da die Temperatur an Ort und Stelle gemessen wird und die abströmenden Dämpfe bzw. deren Druckdampf innerhalb der gesamten Anlage in unmittelbarer Nähe des Gutes gemessen wird. Damit kann auch sichergestellt werden, daß innerhalb des Gutes die gewünschte Temperatur über die vorgegebene Zeit zuverlässig gehalten wird. Zusätzlich kann mit dem Komparator ein Druckmeßfühler verbunden sein, der außerhalb des mikrowellendurchlässigen Behälters in das Innere des Resonanzraumes reicht, wodurch auch die Verhältnisse in dem außerhalb des Behälters befindlichen Bereich des Resonanzraumes ermittelt werden können.

Um zu verhindern, daß sich bei Temperaturdifferenzen an der Wandung des Resonanzraumes Kondensat bildet, kann die Wandung des Resonanzraumes mit einer Heizeinrichtung versehen sein. Für eine besonders genaue Steuerung kann die Leistung der Heizeinrichtung in Abhängigkeit von der im Behälter herrschenden Temperatur regelbar sein.

Um ein gesteuertes Abkühlen und auch eine gesteuerte Druckabsenkung im Resonanzraum zu ermöglichen, können mit der Steuereinrichtung in den Ablaßleitungen vorgesehene Magnetventile verbunden sein.

Schließlich kann der Innenraum des Resonanzraumes und/oder des Druckschlauches der Dichtung mit einem Kompressor und/oder einem Druckspeicher verbunden sein, wodurch ermöglicht wird, den Resonanzraum mit Druck zu beaufschlagen, ohne daß die Bildung der erforderlichen Dampfmenge abgewartet werden muß. Dies hat den Vorteil, daß die entsprechende Temperatur innerhalb der Flüssigkeit ohne Verdampfen derselben vor sich geht, wobei auch in energetischer Beziehung insoferne eine Einsparung erfolgen kann, als aufgrund des von außen eingebrachten Druckes weniger Wasser verdampft werden muß, um den Druck aufzubauen. Es kann damit allfällig erforderliche Verdampfungsenergie eingespart werden. Weiters kann der Druck im Resonanzraum gespeichert und für die nächste Druckbeaufschlagung der Druckdichtung herangezogen werden.

Bei einem vorteilhaften Verfahren zum Pasteurisieren, Desinfizieren/Sterilisieren von feuchten, befeuchteten oder in feuchter Umgebung befindlichen Gütern sowie von Flüssigkeit mittels der erfindungsgemäßen Anlage können die zu behandelnden Güter in einen mikrowellendurchlässigen, wärmebeständigen Behälter eingebracht werden, dieser Behälter dann in den Resonanzraum eingesetzt und gegebenenfalls in diesen Behälter Wasser zugesetzt wird, danach der Resonanzraum druckdicht verschlossen und anschließend der Resonanzraum mit Mikrowellen baufschlagt und die darin befindlichen Güter unter Steigerung des Druckes im Resonanzraum auf die gewünschte Temperatur erwärmt werden, wobei durch Messung der Temperatur des aus den Gütern abströmenden Dampfes die Leistung der Magnetrone geregelt und Temperatur und Druck über die gewünschte Behandlungsdauer einem vorgewählten Muster entsprechend gehalten wird.

Da die gesamte, zur Dampferzeugung verwendete Wassermenge in dem Behälter bzw. in dem zu sterilisierenden Gut ist, bedarf es keinerlei Wasservorlage außerhalb des Behälters im Druckraum und damit auch keinerlei entkalkten Wassers, da allfällig entstehende Rückstände auf den zu entkeimenden bzw. desinfizierenden Gütern verbleiben und mit diesen entsorgt werden. Es kann jedoch auch das zu behandelnde Gut in einem mikrowellendurchlässigen Behälter in den Resonanzraum eingebracht und dieser druckdicht verschlossen werden, wonach dann das zu behandelnde Gut mit Mikrowellen beaufschlagt wird, wobei die Erwärmung des Gutes und damit auch der Trocknungsgrad durch Messung der Temperatur der aus dem Gut abströmenden Dämpfe überwacht wird, und wobei gegebenenfalls der Druckverlauf über den Behandlungszeitraum entsprechend einem vorgewählten Muster über die Leistung der Magnetrone gesteuert wird, womit erreicht wird, daß bei einem druckdichten und druckbeständigen inneren Behälter die hohe Temperatur nur innerhalb des Behälters aufgebracht werden muß, wobei die in den Resonanzraum abströmenden Dämpfe innerhalb des Resonanzraumes von den eingekoppelten Mikrowellen nochmals erhitzt werden und damit eine Kontamination des Resonanzraumes verhindert ist. Weiters kann nach Einbringen des mit Flüssigkeit befüllten Behältnisses und Erhitzung desselben mittels der Mikrowellen die Temperatur und der Druck innerhalb des Behältnisses überwacht werden und der Druck im Resonanzraum außerhalb des Behältnisses mittels einer Druckquelle dem Druck innerhalb des Behältnisses gleich gehalten werden, wobei das Gut durch langsame Druckreduktion innerhalb des Resonanzraumes langsam abgekühlt wird. Damit wird verhindert, daß ein Siedeverzug oder ein sonstiges Überschäumen oder Austreten des Behälterinhalts in den Resonanzraum vermieden ist.

Schließlich kann nach Ende des Sterilisations-/Desinfektionszyklus und Absenken des Druckes im Resonanzraum auf Atmosphärendruck der Resonanzraum und mit diesem der Behälterinnenraum mit Unterdruck beaufschlagt werden, wodurch erreicht wird, daß das desinfizierte oder sterilisierte Gut rasch trocknet und in dieser trockenen Form dann der Weiterverwertung zugeführt werden kann.

In der Zeichnung ist ein Ausführungsbeispiel des Erfindungsgegenstandes dargestellt.

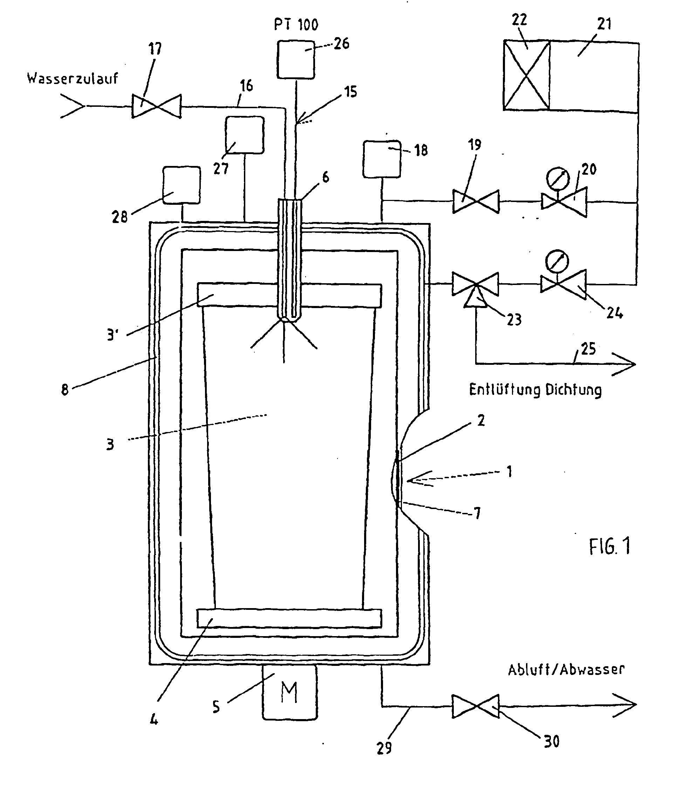

Fig. 1 zeigt die Gesamtanlage schematisch mit angeschlossenen Nebenaggregaten.

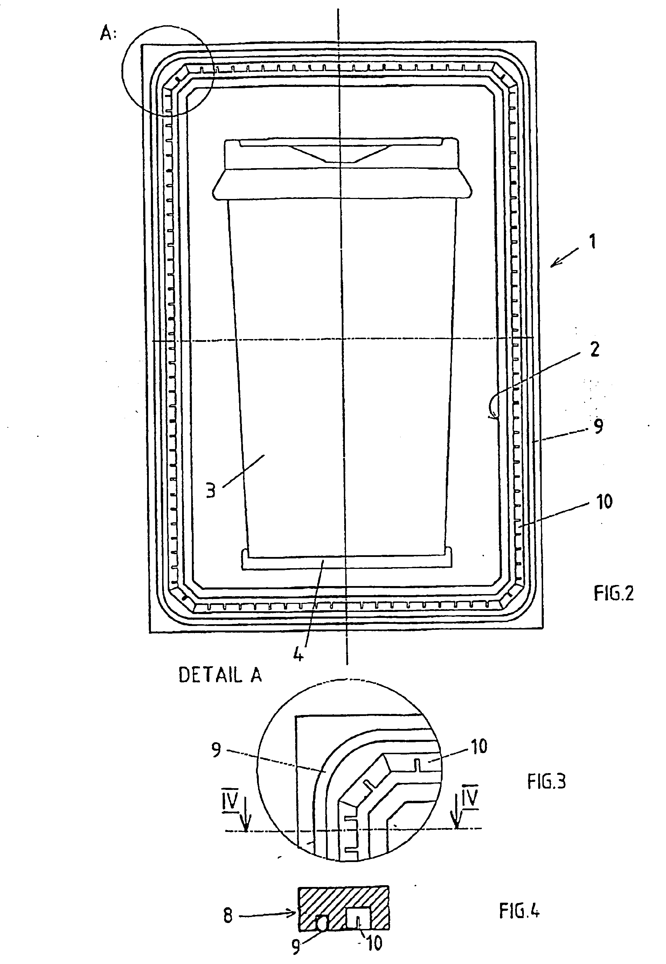

Fig. 2 zeigt, ebenfalls schematisch, den Resonanzraum in Vorderansicht bei geöffneter Beschickungsöffnung.
Fig. 3 zeigt im Detail den Ausschnitt A der Fig. 2.

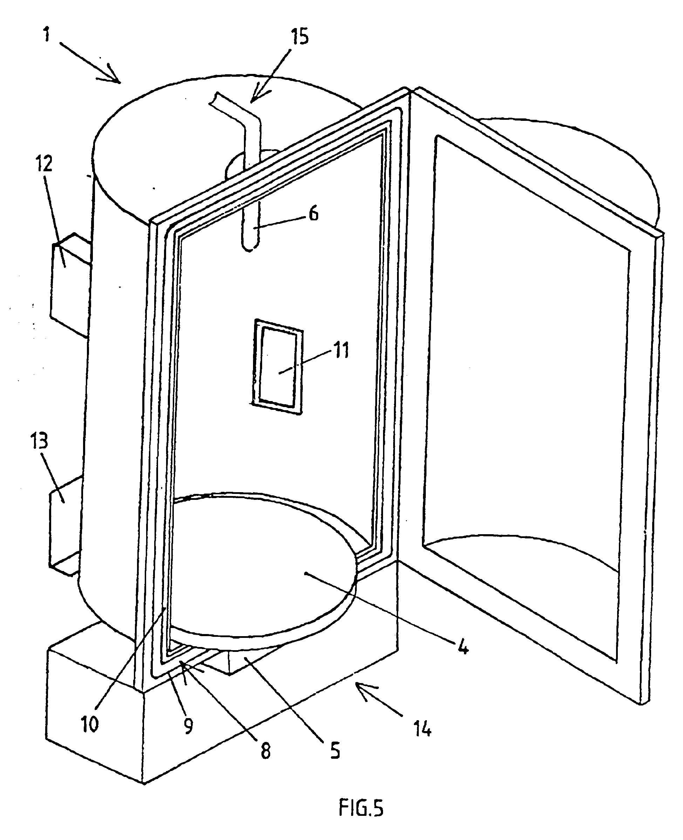

Fig. 4 ist ein Schnitt nach Linie IV-IV der Fig. 3.
Fig. 5 zeigt den Erfindungsgegenstand schematisch im Schaubild.

Mit 1 ist ein Resonanzraum bezeichnet, der durch seine Innenwandung 2 begrenzt ist. In diesen Resonanzraum 1 ist ein Behälter 3 mit Deckel 3' einsetzbar, u.zw. auf einem Drehteller 4, der mit einem Antriebsmotor 5 in Verbindung steht. Dieser Drehteller kann gleichzeitig auch eine Wägeeinrichtung darstellen, mit welcher das Gewicht des eingesetzten gefüllten Behälters 3 ermittelbar ist. Der Deckel 3' weist eine zentrale Öffnung auf, in welcher ein Träger 6 für Meßeinrichtungen mit dem Behälterinneren in Verbindung gebracht werden kann.

Die Innenwandung 2 des Resonanzraumes 1 ist mit einer Heizung 7 versehen, welche im Bedarfsfall zusätzlich zu den Mikrowellengeneratoren zuschaltbar ist. Am Rand der Beschickungsöffnung weist der Behandlungsraum eine Kombination 8 aus Druckdichtung 9 und Wellenfalle 10 auf, wobei die Druckdichtung durch einen Druckschlauch gebildet ist, der nach Schließen der Türe mit Innendruck beaufschlagbar ist und damit einen zuverlässigen druckdichten Abschluß der Beschickungsöffnung ermöglicht.

In Fig. 5 sind mit 11 12, 13 drei Magnetrone angedeutet, die über an sich bekannte Hohlleiter in den Behandlungsraum 1 eingekoppelt sind, wobei das Austrittsfenster für die Mikrowellen mit der Innenwandung 2 druckdicht verschlossen ist.

Wie ebenfalls in Fig. 5 ersichtlich, steht die gesamte Anlage auf einem Traggestell 14, in welchem sich auch der Antriebsmotor 5 für den Drehteller und gegebenenfalls noch zusätzliche Einrichtungen befinden können.

Mit 15 sind die aus dem Träger 6 für Meßeinrichtungen herausführenden Steuerleitungen bezeichnet, wobei der Träger 6 für die Meßeinrichtungen einen Temperatursensor, einen Drucksensor und gegebenenfalls noch zusätzliche Einrichtungen enthalten kann.

In den Träger 6 mündet auch eine Wasserzuleitung 16 ein, die mittels eines Magnetventils 17 abschließbar ist, um - falls erforderlich - in den Behälter 3 zusätzliche Behandlungsmedium oder sonstige Zusätze einzubringen.

Am Resonanzraum 1 ist ein vom Träger 6 gesonderter Drucksensor 18 vorgesehen, von dessen Zuleitung eine ein Magnetventil 19 und einen Druckminderer 20 aufweisende Leitung zu einem Druckgefäß 21 führt, das seinerseits mit einem Kompressor 22 verbunden ist. Das Druckgefäß 21 bzw. der Kompressor 22 ist über eine mit einem Magnetventil 23 und einem Druckminderer 24 versehene Leitung auch mit dem Innenraum der Druckdichtung 9 verbunden. Von dem Magnetventil 23 führt eine Entlüftungsleitung 25 für die Dichtung 9 weg. Über die mit dem Magnetventil 19 versehene Leitung können auch zur Behandlung zweckdienliche gasförmige Medien eingebracht werden.

Der im Träger 6 für die Meßeinrichtung vorgesehene Temperaturmeßfühler ist mit einer Temperaturmeßeinrichtung 26 verbunden.

Mit dem Resonanzraum 1 ist auch noch ein Sicherheitsventil 27 und eine Vakuumpumpe 28 verbunden. Vom Boden des Behandlungsraumes 1 führt noch eine Abluft-/Abwasserleitung 29 weg, in der ein Magnetventil 30 eingeschaltet ist.

Alle Magnet- und Steuereinrichtungen sowie alle Meßfühler sind mit einer gemeinamen, nicht dargestellten Steuereinrichtung verbunden, die die ermittelten Istwerte mit vorgegebenen Sollwerten vergleicht und dann aufgrund dieses Vergleiches die Leistung der Magnetrone 11, 12 und 13 steuert.

Ist nun ein Behälter 3 mit infektiösem Abfall so weit voll, daß die Entsorgung ansteht, dann wird dieser Behälter 3 durch den Deckel 3' verschlossen und auf den Drehteller 4 des Behandlungsraumes 1 eingesetzt. Danach wird in die Steuereinrichtung die Art des angefallenen Abfalles eingegeben, wodurch dann die Steuereinrichtung aufgrund der über den Drehteller 4 erfaßten Wägedaten die allfällig erforderliche Wassermenge nach einem vorgegebenen Algorithmus berechnet. Dieser Algorithmus und die Berechnung und Steuerung der Wasserzugabe ist in der eingangs genannten EP 0 483 104 B1 ausführlich dargelegt, auf welche Druckschrift hier ausdrücklich Bezug genommen wird und deren Inhalt damit in vorliegende Anmeldung durch Zitierung aufgenommen wird.

Nach Zugabe der erforderlichen Wassermenge über die Leitung 16 durch Steuerung des Magnetventils 17 mittels der zentralen Steuereinrichtung und Schließen des Tores sowie Druckbeaufschlagung der Druckdichtung 9 werden die Magnetrone 11, 12, 13 mit Energie beaufschlagt, wodurch es zu einer Erwärmung der Flüssigkeit im Behälter 3 kommt. Falls die Temperaturdifferenz zwischen Innenwandung 2 des Resonanzraumes 1 und den aus dem Behälter austretenden Dämpfen zu groß ist, wird die Heizeinrichtung 7 eingeschaltet, um damit die Temperatur der Wandung an die Temperatur der Dämpfe anzupassen und ein Kondensieren zu verhindern. Aufgrund der Distanz der Magnetrone vom Behandlungsraum und die Einkopplung über die Hohlleiter werden die im Behandlungsraum 1 befindlichen Dämpfe und damit auch die Innenwandung 2 des Behandlungsraumes so stark beheizt, daß eine gesonderte Beheizung der Innenwandung 2 fast nicht nötig ist. Mittels des am Träger 6 befindlichen, in den Innenraum des Behälters 3 eingebrachten Temperaturmeßfühlers wird die Temperatur der Dämpfe und damit auch die Temperatur des im Behälter 3 befindlichen Behandlungsgutes gemessen, wodurch indirekt auch eine Druckmessung erfolgt. Da die Druckmessung jedoch aufgrund der in den Abfällen vorhandenen flüchtigen Substanzen verfälscht sein kann, wird parallel der Druck im Behandlungsraum 1 auch über den Drucksensor 18 gemessen, wodurch auch ermittelt werden kann, ob die flüchtigen Substanzen bereits verdampft sind und innerhalb des Behandlungsraumes 1 nur mehr reiner Wasserdampf vorliegt oder ob darin noch Dämpfe flüchtiger Substanzen vorhanden sind. Wesentlich ist die Einhaltung der erforderlichen Temperatur innerhalb des Behälters 3 über einen vorgegebenen Zeitraum, da nur dadurch sichergestellt werden kann, daß eine zuverlässige Abtötung aller Keime bzw. auch Sporen erfolgt ist.

Nach Beendigung des Behandlungszyklus wird der Druck innerhalb des Behandlungsraumes 1 über das Magnetventil 19 , über den Druckmesser 20 und über ein Druckgefäß 21 gesteuert abgelassen, um Siedeverzüge oder sonstige eruptive Ausbrüche innerhalb des Gefäßes 3 zu verhindern. Sollte am Ende des Behandlungszeitraumes das Gut noch zu feucht sein, dann kann nach Erreichen des Normaldruckes über die Vakuumpumpe 28 noch eine Vakuumtrocknung nachgeschaltet werden, was insoferne problemlos ist, als der Inhalt des Behälters 3 und auch der Innenraum der Behandlungskammer 1 steril ist, sodaß sich die Verwendung von Sterilfiltem bei der Vakuumpumpe erübrigt.

Sobald auch dann der Normaldruck innerhalb der Behandlungskammer 1 wiederhergestellt ist, wird aus dem Druckschlauch der Dichtung 9 der Druck über das Magnetventil 23 und die Leitung 25 abgelassen, wonach dann das Gefäß 3 samt Deckel 3' aus der Behandlungskammer 1 herausgenommen und der Inhalt des Gefäßes 3 der normalen Entsorgung zugeleitet werden kann.

## Patentansprüche

1. Anlage zum Trocknen, Erhitzen, Sterilisieren und/oder Desinfizieren von feuchten, befeuchteten oder in feuchter Umgebung befindlichen Gütern sowie von Flüssigkeiten, insbesondere von Abfällen, die gegebenenfalls infiziert sind, z.B. von medizinischen Abfällen, bei welcher die zu behandelnden Güter in einem mikrowellendurchlässigen Behälter in einen mit Mikrowellen beaufschlagbaren, druckdicht abschließbaren, druckfesten Behandlungsraum eingebracht werden, wobei mehrere Magnetrone so in den den Behandlungsraum bildenden Resonanzraum eingekoppelt sind, daß mikrowellentote Räume vermieden sind, wobei die physikalischen Parameter im Gerät über an mit dem Behälterinnenraum kommunizierenden Trägern angeordneten Einrichtungen überwacht werden, und gegebenenfalls zur Steuerung der Anlage herangezogen werden, und wobei noch Zusatzaggregate zur Beeinflussung des Verfahrensablaufes vorgesehen sind, **dadurch gekennzeichnet, daß** die Magnetrone (11, 12, 13) druckdicht über an sich bekannte Hohlleiter von der Behälterseitenwandung her in den Behandlungsraum (1) eingekoppelt sind, der mit einer seitlichen Beschickungsöffnung zum Einbringen des mikrowellendurchlässigen Behälters (3) unter Belassung eines Freiraumes zwischen Behälter (3) und Behandlungsrauminnenwandung (2) versehen ist, wobei der Behandlungsraum (1) zylindrische oder polygonal prismatische Form mit vertikal verlaufender Längsachse aufweist, und wobei die Beschickungsöffnung durch Teilen der Behandlungskammer (1) entlang einer parallel zur Längsachse der Behandlungskammer (1) verlaufende, vorzugsweise außermittige Ebene, erzeugt ist, wobei auch die Boden- und die Deckwandung geteilt ist, oder durch Ausschneiden der Mantelwandung der Behandlungskammer (1) entlang zweier Erzeugenden sowie parallel zur Boden- und Deckwandung erzeugt ist.

2. Anlage nach Anspruch 1, **dadurch gekennzeichnet, daß** entlang der Beschickungsöffnung zum dichten Abschluß dieser Öffnung mittels des zugehörigen Abschlußteiles eine Kombination (8) aus an sich bekannter Wellenfalle (10) und Dichtung (9), vorzugsweise eine mit Innendruck beaufschlagbare Schlauchdichtung, vorgesehen ist.

3. Anlage nach Anspruch 1 oder 2, **dadurch gekennzeichnet, daß** zur Bestimmung der Regelgröße für die Leistung der Magnetrone (11, 12, 13) ein Thermofühler (6) über den mit dem Behälterinnenraum kommunizierenden Träger (10) in den mikrowellendurchlässigen Behälter (3) einbringbar ist, der mit einer, einen Sollwertspeicher und einen Komparator zum Vergleich mit dem erhobenen Istwert aufweisenden Steuereinrichtung verbunden ist.

4. Anlage nach einem der Ansprüche 1 bis 3, **dadurch gekennzeichnet, daß** mit dem Komparator ein Druckmeßfühler (18) verbunden ist, der außerhalb des mikrowellendurchlässigen Behälters (3) in das Innere des Resonanzraumes (1) reicht.

5. Anlage nach einem der Ansprüche 1 bis 4, **dadurch gekennzeichnet, daß** die Wandung (2) des Resonanzraumes (1) mit einer Heizeinrichtung (7) versehen ist.

6. Anlage nach Anspruch 5, **dadurch gekennzeichnet, daß** die Leistung der Heizeinrichtung (7) in Abhängigkeit von der im Behälter (3) herrschenden Temperatur regelbar ist.

7. Anlage nach einem der Ansprüche 1 bis 5, **dadurch gekennzeichnet, daß** mit der Steuereinrichtung in den Ablaßleitungen vorgesehene Magnetventile (19, 30) verbunden sind.

8. Anlage nach einem der Ansprüche 1 bis 7, **dadurch gekennzeichnet, daß** der Innenraum des Resonanzraumes (1) und/oder der Dichtung (9) mit einem Kompressor (22) und/oder einem Druckspeicher (21) verbunden ist.

9. Verfahren zum Pateurisieren, Desinfizieren/Sterilisieren von feuchten, befeuchteten oder in feuchter Umgebung befindlichen Gütern sowie von Flüssigkeiten mit einer Anlage nach einem der Ansprüche 1 bis 8, **dadurch gekennzeichnet, daß** die zu behandelnden Güter in einen mikrowellendurchlässigen, wärmebeständigen Behälter eingebracht werden, dieser Behälter dann in den Resonanzraum eingesetzt und gegebenenfalls in diesen Behälter Wasser oder sonstige Behandlungsmedien zugesetzt werden, danach der Resonanzraum druckdicht verschlossen und anschließend der Resonanzraum mit Mikrowellen baufschlagt und die darin befindlichen Güter unter Steigerung des Druckes im Resonanzraum auf die gewünschte Temperatur erwärmt werden, wobei durch Messung der Temperatur des aus den Gütern abströmenden Dampfes die Leistung der Magnetrone geregelt und Temperatur und Druck über die gewünschte Behandlungsdauer einem vorgewählten Muster entsprechend gehalten werden.

10. Verfahren zum Trocknen von feuchten, befeuchteten oder in feuchter Umgebung befindlichen Gütern sowie von Flüssigkeiten in einer Anlage nach einem der Ansprüche 1 bis 8, **dadurch gekennzeichnet, daß** das zu behandelnde Gut in einem mikrowellendurchlässigen Behälter in den Resonanzraum eingebracht und dieser druckdicht verschlossen wird, wonach dann das zu behandelnde Gut mit Mikrowellen beaufschlagt wird, wobei die Erwärmung des Gutes und damit auch der Trocknungsgrad durch Messung der Temperatur der aus dem Gut abströmenden Dämpfe überwacht wird, und wobei gegebenenfalls der Druckverlauf über den Behandlungszeitraum entsprechend einem vorgewählten Muster über die Leistung der Magnetrone gesteuert wird.

11. Verfahren zur Sterilisation von Flüssigkeiten in nicht druckbeständigen geschlossenen Behältnissen in einer Anlage nach Anspruch 7, **dadurch gekennzeichnet, daß** nach Einbringen des mit Flüssigkeit befüllten Behältnisses und Erhitzung desselben mittels der Mikrowellen die Temperatur und/oder der Druck innerhalb des Behältnisses überwacht wird und der Druck im Resonanzraum außerhalb des Behältnisses mittels einer Druckquelle, z.B. unter Einbringen von Behandlungsgas, dem Druck innerhalb des Behältnisses gleich gehalten wird, wobei das Gut durch langsame Druckreduktion innerhalb des Resonanzraumes langsam abgekühlt wird.

12. Verfahren nach einem der Ansprüche 9 bis 11, **dadurch gekennzeichnet, daß** nach Ende des Sterilisations-/Desinfektionszyklus und Absenken des Druckes im Resonanzraum auf Atmosphärendruck der Resonanzraum und mit diesem der Behälterinnenraum mit Unterdruck beaufschlagt wird.

## Claims

1. An installation for the drying, heating, sterilisation and/or disinfection of articles which are moist, moistened or situated in a moist environment, and of liquids, particularly of waste, which is potentially infected, e.g. of medicinal waste, in which the articles to be treated are introduced in a container which is permeable to microwaves, into a pressure-tight, sealable, pressure-resistant treatment space, to which microwaves may be applied, wherein a plurality of magnetrons are so coupled into the resonance space constituting the treatment space that regions free of microwaves are prevented, whereby the physical parameters in the device are monitored by means of devices arranged on carriers communicating with the interior of the container and are optionally used to control the installation, and wherein additional units are also provided to influence the progress of the process, **characterised in that** the magnetrons (11, 12, 13) are coupled in a pressure-tight manner by means of hollow conductors known per se from the container side wall into the treatment space (1), which is provided with a lateral loading opening for the introduction of the container (3), which is permeable to microwaves, whilst leaving an empty space between the container (3) and the inner wall of the treatment space, wherein the treatment space (1) has a cylindrical or polygonal prismatic shape with a vertically extending longitudinal axis and wherein the loading opening is produced by dividing the treatment chamber (1) along a preferably off centre plane extending parallel to the longitudinal axis of the treatment chamber (1), the base and top wall also being divided or being generated by cutting out the casing wall of the treatment chamber (1) along two generatrices and parallel to the base and top wall.

2. An installation as claimed in Claim 1, **characterised in that** a combination (8) of a wave trap (10) known per se and a seal (9), preferably an internally pressurisable hose seal, is provided along the loading opening for the sealed closure of this opening by means of the associated closure portion.

3. An installation as claimed in Claim 1 or 2, **characterised in that** in order to determine the control parameter for the output of the magnetrons (11, 12, 13), a temperature sensor (6) may be introduced into the container (3) permeable to microwaves by means of the carrier (10) communicating with the interior of the container, which sensor is connected to a control device including a desired value store and a comparator for comparing with the raised actual value.

4. An installation as claimed in one of Claims 1 to 3, **characterised in that** connected to the comparator there is a pressure measuring sensor (18), which extends outside the container (3) permeable to microwaves into the interior of the resonance space (1).

5. An installation as claimed in one of Claims 1 to 4, **characterised in that** the wall (2) of the resonance space (1) is provided with a heating device (7).

6. An installation as claimed in Claim 5, **characterised in that** the output of the heating device (7) is controllable in dependence on the temperature prevailing in the container (3).

7. An installation as claimed in one of Claims 1 to 5, **characterised in that** magnetic valves (19, 30) provided in the discharge lines are connected to the control device.

8. An installation as claimed in one of Claims 1 to 7, **characterised in that** the interior of the resonance space (1) and/or of the seal (9) is connected to a compressor (22) and/or a pressure reservoir (21).

9. A process for the pasteurisation, disinfection/sterilisation of articles which are moist, moistened or situated in a moist environment, and of liquids with an installation as claimed in one of Claims 1 to 8, **characterised in that** the articles to be treated are introduced into a heat-resistant container permeable to microwaves, this container is then placed in the resonance space and water or other treatment media are optionally added into this container, whereafter the resonance space is closed in a pressure-tight manner and microwaves are then applied to the resonance space and the articles situated in it are heated to the desired temperature whilst increasing the pressure in the resonance space, wherein the output of the magnetrons is controlled by measurement of the temperature of the steam escaping from the articles and the temperature and pressure are maintained in accordance with a predetermined pattern over the desired treatment period.

10. A process for the drying of articles which are moist, moistened or situated in a moist environment and of liquids in an installation as claimed in one of Claims 1 to 8, **characterised in that** the article to be treated is introduced into a container permeable to microwaves and it is then closed in a pressure-tight manner, whereafter the article to be treated is then subjected to microwaves, wherein the heating of the article and thus also the degree of drying is monitored by measuring the temperature of the steam escaping from the article and wherein optionally the variation in pressure over the period of treatment is controlled in accordance with a predetermined pattern by means of the output of the magnetrons.

11. A process for the sterilisation of liquids in closed containers which are not pressure-resistant in an installation as claimed in Claim 7, **characterised in that** after introducing the container filled with liquid and heating of the same by means of the microwaves, the temperature and/or the pressure within the container is monitored and the pressure in the resonance space outside the container is maintained equal to the pressure within the container by means of a pressure source, e.g. with the introduction of treatment gas, whereby the article is slowly cooled by a slow reduction in pressure within the resonance space.

12. A process as claimed in one of Claims 9 to 11, **characterised in that** after the end of the sterilisation/disinfection cycle and reduction of the pressure in the resonance space to atmospheric pressure, a reduced pressure is applied to the resonance space and thus the interior of the container.

## Revendications

1. Installation de séchage, chauffage, stérilisation et/ou désinfection de produits humides, humidifiés ou situés dans un environnement humide, ainsi que de liquides, en particulier des déchets qui, le cas échéant, sont infectés, tels que des déchets médicaux, dans laquelle installation les produits à traiter, contenus dans un récipient perméable aux micro-ondes, sont introduits à l'intérieur d'une chambre de traitement résistante à la pression, pouvant être sollicitée par des micro-ondes, pouvant être fermée de manière étanche à la pression, plusieurs magnétrons étant couplés à l'intérieur de la chambre de résonance formant la chambre de traitement, de telle sorte que des espaces exempts de micro-ondes sont évités, les paramètres physiques dans l'appareil étant contrôlés par l'intermédiaire de dispositifs agencés sur des supports communiquant avec la chambre de traitement et étant utilisés, le cas échéant, pour la commande de l'installation, et des organes supplémentaires étant prévus pour influer sur le déroulement du procédé, **caractérisée en ce que** les magnétrons (11, 12, 13) sont couplés, de manière étanche à la pression par l'intermédiaire de conducteurs creux connus en soi, à partir de la paroi latérale du récipient vers l'intérieur de la chambre de traitement (1), qui est munie d'une ouverture de chargement latérale pour introduire le récipient (3) perméable aux micro-ondes en laissant un intervalle entre le récipient (3) et la paroi intérieure (2) de la chambre de traitement, la chambre de traitement (1) ayant une forme cylindrique ou polygonale prismatique avec un axe longitudinal orienté verticalement, et l'ouverture de chargement étant formée par une division de la chambre de traitement (1) le long d'un plan, de préférence excentré, parallèle à l'axe longitudinal de la chambre de traitement (1), la paroi inférieure et la paroi supérieure étant également divisées ou étant formées par découpe de la paroi latérale de la chambre de traitement (1) le long de deux génératrices, ainsi que parallèlement à la paroi inférieure et à la paroi supérieure.

2. Installation selon la revendication 1, **caractérisée en ce que** le long de l'ouverture de chargement, en vue de la fermeture étanche de cette ouverture au moyen de la partie d'obturation associée, il est prévu une combinaison (8) formée par des pièges d'ondes (10), connus en soi, et une garniture d'étanchéité (9), de préférence une garniture d'étanchéité en forme de tuyau flexible apte à être sollicitée par une pression intérieure.

3. Installation selon la revendication 1 ou 2, **caractérisée en ce que**, pour déterminer la dimension standard pour le rendement des magnétrons (11, 12, 13), une sonde thermique (6) peut être introduite, par l'intermédiaire du support (10) communiquant avec l'intérieur du récipient, dans le récipient (3) perméable aux micro-ondes, qui est relié à un dispositif de commande comportant une mémoire de valeurs de consigne et un comparateur en vue d'une comparaison avec la valeur réelle élevée.

4. Installation selon l'une quelconque des revendications 1 à 3, **caractérisée en ce qu'**au comparateur est relié un capteur de pression (18), qui à l'extérieur du récipient (3) perméable aux micro-ondes s'engage à l'intérieur de la chambre de résonance (1).

5. Installation selon l'une quelconque des revendications 1 à 4, **caractérisée en ce que** la paroi (2) de la chambre de résonance (1) est munie d'un dispositif de chauffage (7).

6. Installation selon la revendication 5, **caractérisée en ce que** le rendement du dispositif de chauffage (7) peut être réglé en fonction de la température qui règne dans le récipient (3).

7. Installation selon l'une quelconque des revendications 1 à 5, **caractérisée en ce que** des électrovannes (19, 30), prévues dans les conduites de sortie, sont reliées au dispositif de commande.

8. Installation selon l'une quelconque des revendications 1 à 7, **caractérisée en ce que** l'intérieur de la chambre de résonance (1) et/ou de la garniture d'étanchéité (9) est relié à un compresseur (22) et/ou un réservoir de pression (21).

9. Procédé de pasteurisation, désinfection et/ou stérilisation de produits humides, humidifiés ou situés dans un environnement humide, ainsi que de liquides, avec une installation selon l'une quelconque des revendications 1 à 8, **caractérisé en ce que** les produits à traiter sont introduits dans un récipient perméable aux micro-ondes et résistant à la chaleur, ce récipient est posé ensuite dans la chambre de résonance et, le cas échéant, de l'eau ou d'autres produits de traitement sont ajoutés dans ce récipient, puis la chambre de résonance est fermée de manière étanche à la pression et ensuite la chambre de résonance est sollicitée par des micro-ondes et les produits contenus dans celle-ci sont chauffés à la température souhaitée moyennant l'augmentation de la pression dans la chambre de résonance, le rendement des magnétrons étant régulé au moyen de la mesure de la température de la vapeur sortant des produits, et la température et la pression étant maintenues conformément à un modèle prédéfini sur toute la durée de traitement souhaitée.

10. Procédé de séchage de produits humides, humidifiés ou situés dans un environnement humide, ainsi que de liquides, avec une installation selon l'une quelconque des revendications 1 à 8, **caractérisé en ce que** les produits à traiter, contenus dans un récipient perméable aux micro-ondes, sont introduits dans la chambre de résonance et celle-ci est fermée de manière étanche à la pression, à la suite de quoi le produit à traiter est sollicité par des micro-ondes, le chauffage du produit et donc aussi le degré de séchage étant surveillés au moyen de la mesure de la température de la vapeur sortant des produits, et, le cas échéant, l'évolution de la pression pendant la durée de traitement étant commandée par l'intermédiaire du rendement des magnétrons conformément à un modèle prédéfini.

11. Procédé de stérilisation de liquides dans des récipients fermés non résistants à la pression dans une installation selon la revendication 7, **caractérisé en ce que**, après l'introduction du récipient rempli de liquide et le chauffage de celui-ci au moyen des micro-ondes, la température et/ou la pression sont surveillées à l'intérieur du récipient, et la pression dans la chambre de résonance à l'extérieur du récipient est maintenue au moyen d'une source de pression, par exemple par l'introduction de gaz de traitement, au même niveau que la pression à l'intérieur du récipient, le produit étant lentement refroidi par une lente diminution de la pression à l'intérieur de la chambre de résonance.

12. Procédé selon l'une quelconque des revendications 9 à 11, **caractérisé en ce que**, à l'issue du cycle de stérilisation et de désinfection et de la diminution de la pression dans la chambre de résonance jusqu'à la pression atmosphérique, la chambre de résonance et, conjointement avec celle-ci, l'intérieur du récipient sont sollicités par une dépression.
